# EUROPEAN PATENT SPECIFICATION

(11) **EP 0 725 788 B1**
(45) Date of publication and mention of the grant of the patent: **16.12.1998**
(21) Application number: 94932003.0
(22) Date of filing: 24.10.1994
(51) Int. Cl.: C07H 21/00

(54) **2'-AMIDO AND 2'-PEPTIDO MODIFIED OLIGONUCLEOTIDES**
2'-AMIDO-UND 2'-PEPTIDO-MODIFIZIERTE OLIGONUKLEOTIDE
2'-AMIDO ET 2'-PEPTIDO OLIGONUCLEOTIDES MODIFIES

(30) Priority: 27.10.1993 US 143832
(43) Date of publication of application: 14.08.1996
(73) Proprietor: RIBOZYME PHARMACEUTICALS, INC., Boulder, CO 80301 (US)
(72) Inventor: DUDYCZ, Lech, W., Boulder, CO 80301 (US)
(74) Representative: Moon, Donald Keith
(86) International application number: PCT/US94/12164
(87) International publication number: WO 95/11910

(56) References cited:
- WO-A-88/00201
- WO-A-91/06556
- JOURNAL OF CARBOHYDRATES NUCLEOSIDES AND NUCLEOTIDES, vol.7, no.5, 1980 pages 297 - 313 S.CHLADEK ET AL. 'Aminoacyl Derivatives of Nucleosides, Nucleotides and Polynucleotides. XXXII. Synthesis of Aminoacyldinucleoside Phosphates Derived from 2'- and 3'-Aminodeoxyadenosine.'

## Description

This invention relates to 2' - modifications of oligonucleotides.

Usman et al., "Nucleozymes", International Application No. WO93/15187 (PCT/US93/0833), describe modification of the 2'-hydroxyl group of RNA to produce modified nucleotides. Such nucleotides are termed nucleic acid analogues, and may have a "good coordinating ligand" with divalent metal ions, e.g., a halogen, or amine group. Acyclic analogues are also proposed.

Eckstein, International Application No. WO 92/07065 (PCT/EP91/0811), describes 2'-hydroxyl modifications of RNA having the following substitutions in place of the hydroxyl group: halo, sulfhydryl, azido, amino, monosubstituted amino and disubstituted amino.

WO 91/06556 describes oligomers having 2' modifications including substitution of the 2' hydroxyl with aminoacyl groups.

WO 88/00201 describes oligomers having 2' modifications including substituted amino.

Chladek et. al., J. Carb. Nucs. Nuct. 7(5), 297-313, 1980, describes oligomers having 2' modifications including glycine and glutamine substituted amino.

### Summary of the Invention

This invention relates to replacement of the 2'-hydroxyl group of one or more ribonucleotide moieties within an oligonuceltodide with a 2'-amido or 2'-peptido moiety. In one aspect the invention provides the use of an oligonucleotide having the general structure shown in Formula 1 below: 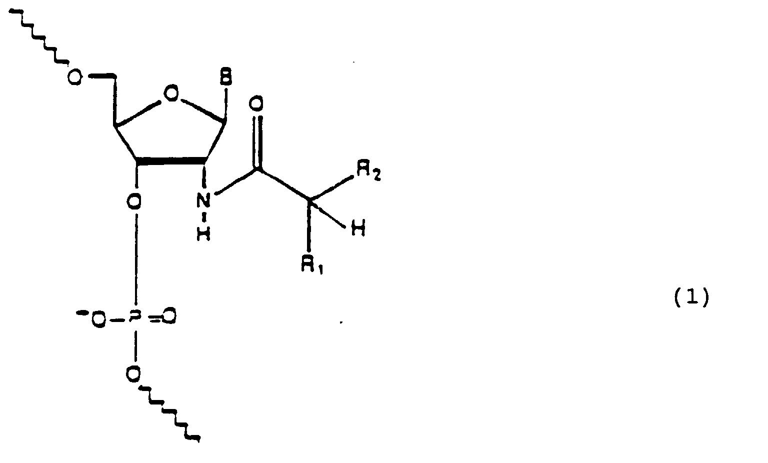 wherein B is a nucleotide base or hydrogen; R₁ and R₂ independently is selected from the group consisting of hydrogen, an alkyl group containing between 2 and 10 carbon atoms inclusive, an amine, an amino acid, and a peptide containing between 2 and 5 amino acids inclusive; and the zigzag lines are independently hydrogen or a bond in the manufacture of a medicament for cleavage of a target gene.

In another aspect the invention provides an oligonucleotide of formula (1) wherein B, R₁ and R₂ and the zigzag lines have the same meaning as above provided that when B is uracil R₁ and R₂ are not both H or when B is adenine and one of R₁ and R₂ is NH₂ the other is not CH₂-CH₂-COOH or when B is uracil or adenine one of R₁ and R₂ is NH₂ the other is not H.

The base (B) is any one of the standard bases or is a modified nucleotide base known to those in the art, or can be a hydrogen group. In addition, either R₁ or R₂ is H or an alkyl group containing between 2 and 10 carbon atoms, an amine (primary, secondary or tertiary, e.g., R₃NR₄ where each R₃ and R₄ independently is hydrogen or an alkyl, alkene or alkyne having between 2 and 10 carbon atoms, or is a residue of an amino acid, i.e., an amide), an amino acid (D or L forms) or peptide containing between 2 and 5 amino acids. The zigzag lines represent hydrogen, or a bond to another base or other chemical moiety known in the art. Preferably, one of R₁ and R₂ is an H, and the other is an amino acid or peptide.

Applicant has recognized that RNA can assume a much more complex structural form than DNA because of the presence of the 2'-hydroxyl group in RNA. This group is able to provide additional hydrogen bonding with other hydrogen donors, acceptors and metal ions within the RNA molecule. Applicant now provides molecules which have a modified amine group at the 2' position, such that significantly more complex structures can be formed by the modified oligonucleotide. Such modification with a 2'-amido or peptido group leads to expansion and enrichment of the side-chain hydrogen bonding network. The amide and peptide moieties are responsible for complex structural formation of the oligonucleotide and can form strong complexes with other bases, and interfere with standard base pairing interactions. Such interference will allow the formation of a complex nucleic acid and protein conglomerate.

Oligonucleotides of this invention are significantly more stable than existing oligonucleotides and can potentially form biologically active bioconjugates not previously possible for oligonucleotides. They may also be used for in vitro selection of unique aptamers, that is, randomly generated oligonucleotides which can be folded into an effective ligand for a target protein, nucleic acid or polysaccharide.

Thus, in a first aspect, the invention features an oligonucleotide containing the modified base shown in Formula I, above.

Other features and advantages of the invention will be apparent from the following description of the preferred embodiments thereof, and from the claims.

### Description of the Preferred Embodiments

Oligonucleotides of this invention are described generally above, and the structure is shown in Formula I, where such modifications to the 2'-hydroxyl group can be made in one or more positions of an RNA or DNA molecule. Preferably, the oligonucleotide is single-stranded and has between 10 and 50 bases of which one or more may be modified as shown, preferably, between 1 and 10 are modified. Such oligonucleotides may include those having enzymatic activity, i.e., ribozymes, which are modified in the 2'- position of the sugar moiety as shown in Formula I to provide stability to that enzymatic activity without significant alteration of the activity.

Oligonucleotides of the present invention can be readily synthesized using carbamate protecting groups, such as F-moc, in the peptide moieties and deprotected under mild basic conditions. Such nucleotides can then be incorporated by standard solid phase synthesis using nucleoside phosphoramidite or H-phosphonate intermediates.

### Use

The above nucleotides are particularly useful in ribozymes. Ribozymes are RNA molecules having an enzymatic activity which is able to repeatedly cleave other separate RNA molecules in a nucleotide base sequence specific manner. Such enzymatic RNA molecules can be targeted to virtually any RNA transcript, and efficient cleavage achieved *in vitro.* Kim et al., 84 Proc. Natl. Acad. Sci. USA 8788, 1987; Haseloff and Gerlach, 334 Nature 585, 1988; Cech, 260 J. Am. Med. Soc. 3030, 1988; and Jefferies et al., Nucleic Acids Research 1371, 1989.

Ribozymes act by first binding to a target RNA. Such binding occurs through the target RNA binding portion of a ribozyme which is held in close proximity to an enzymatic portion of the RNA which acts to cleave the target RNA. Thus, the ribozyme first recognizes and then binds a target RNA through complementary base-pairing, and once bound to the correct site, acts enzymatically to cut the target RNA. Strategic cleavage of such a target RNA will destroy its ability to direct synthesis of an encoded protein. After a ribozyme has bound and cleaved its RNA target it is released from that RNA to search for another target and can repeatedly bind and cleave new targets.

The enzymatic nature of a ribozyme is advantageous over other technologies, such as antisense technology (where a nucleic acid molecule simply binds to a nucleic acid target to block its translation) since the effective concentration of ribozyme necessary to effect a therapeutic treatment is lower than that of an antisense oligonucleotide. This advantage reflects the ability of the ribozyme to act enzymatically. Thus, a single ribozyme molecule is able to cleave many molecules of target RNA. In addition, the ribozyme is a highly specific inhibitor, with the specificity of inhibition depending not only on the base pairing mechanism of binding, but also on the mechanism by which the molecule inhibits the expression of the RNA to which it binds. That is, the inhibition is caused by cleavage of the RNA target and so specificity is defined as the ratio of the rate of cleavage of the targeted RNA over the rate of cleavage of non-targeted RNA. This cleavage mechanism is dependent upon factors additional to those involved in base pairing. Thus, it is thought that the specificity of action of a ribozyme is greater than that of antisense oligonucleotide binding the same RNA site.

By "enzymatic RNA molecule" it is meant an RNA molecule which has complementarity in a substrate binding region to a specified gene target, and also has an enzymatic activity which is active to specifically cleave RNA in that target. That is, the enzymatic RNA molecule is able to intermolecularly cleave RNA and thereby inactivate a target RNA molecule. This complementarity functions to allow sufficient hybridization of the enzymatic RNA molecule to the target RNA to allow the cleavage to occur. One hundred percent complementarity is preferred, but complementarity as low as 50-75% may also be useful in this invention. By "equivalent" RNA to picornavirus is meant to include those naturally occurring RNA molecules associated with viral caused diseases in various animals, including humans, and other primates. These viral RNAs have similar structures and equivalent genes to each other.

In preferred embodiments the enzymatic RNA molecule is formed in a hammerhead motif, but may also be formed in the motif of a hairpin, hepatitis delta virus, group I intron or RNaseP RNA (in association with an RNA guide sequence). Examples of such hammerhead motifs are described by Rossi et al., 8 Aids Research and Human Retroviruses 183, 1992; of hairpin motifs in EP-A-0360257, Hampel and Tritz, 28 Biochemistry 4929, 1989 and Hampel et al., 18 Nucleic Acids Research 299, 1990; and an example of the hepatitis delta virus motif is described by Perrotta and Been, 31 Biochemistry 16, 1992; of the RNaseP motif by Guerrier-Takada et al., 35 Cell 849, 1983; and of the group I intron by Cech et al., U.S. Patent 4,987,071. These specific motifs are not limiting in the invention and those skilled in the art will recognize that all that is important in an enzymatic RNA molecule of this invention is that it has a specific substrate binding site which is complementary to one or more of the target gene RNA regions, and that it have nucleotide sequences within or surrounding that substrate binding site which impart an RNA cleaving activity to the molecule.

### Synthesis of Ribozymes

Ribozymes useful in this invention can be produced by chemical synthesis. Chemical synthesis of RNA is similar to that for DNA synthesis. The additional 2'-OH group in RNA, however, requires a different protecting group strategy to deal with selective 3'-5' internucleotide bond formation, and with RNA susceptibility to degradation in the presence of bases. The recently developed method of RNA synthesis utilizing the t-butyldimethylsilyl group for the protection of the 2'-hydroxyl is the most reliable method for synthesis of ribozymes. The method reproducibly yields RNA with the correct 3'-5' internucleotide linkages, with average coupling yields in excess of 99%, and requires only a two-step deprotection of the polymer.

A method based upon H-phosphonate chemistry gives a relatively lower coupling efficiency than a method based upon phosphoramidite chemistry. This is a problem for synthesis of DNA as well. A promising approach to scale-up of automatic oligonucleotide synthesis has been described recently for the H-phosphonates. A combination of a proper coupling time and additional capping of "failure" sequences gave high yields in the synthesis of oligodeoxynucleotides in scales in the range of 14 µmoles with as little as 2 equivalents of a monomer in the coupling step. Another alternative approach is to use soluble polymeric supports (e.g., polyethylene glycols), instead of the conventional solid supports. This method can yield short oligonucleotides in hundred milligram quantities per batch utilizing about 3 equivalents of a monomer in a coupling step.

Various modifications to ribozyme structure can be made to enhance the utility of ribozymes. Such modifications will enhance shelf-life, half-life *in vitro,* stability, and ease of introduction of such ribozymes to the target site, e.g., to enhance penetration of cellular membranes, and confer the ability to recognize and bind to targeted cells.

Exogenous delivery of ribozymes benefits from chemical modification of the backbone, e.g., by the overall negative charge of the ribozyme molecule being reduced to facilitate diffusion across the cell membrane. The present strategies for reducing the oligonucleotide charge include: modification of internucleotide linkages by ethylphosphonates, use of phosphoramidites, linking oligonucleotides to positively charged molecules, and creating complex packages composed of oligonucleotides, lipids and specific receptors or effectors for targeted cells. Examples of such modifications include sulfur-containing ribozymes containing phosphorothioates and phosphorodithioates as internucleotide linkages in RNA. Synthesis of such sulfur-modified ribozymes is achieved by use of the sulfur-transfer reagent, ³H-1,2-benzenedithiol-3-one 1,1-dioxide. Ribozymes may also contain ribose modified ribonucleotides as described herein. Ribozymes can also be either electrostatically or covalently attached to polymeric cations for the purpose of reducing charge. The polymer can be attached to the ribozyme by simply converting the 3'-end to a ribonucleoside dialdehyde which is obtained by a periodate cleavage of the terminal 2',3'-cis diol system. Depending on the specific requirements for delivery systems, other possible modifications may include different linker arms containing carboxyl, amino or thiol functionalities. Yet further examples include use of methylphosphonates and 2'-O-methylribose and 5' or 3' capping or blocking with m₇GpppG or m₃^{2,2,7}GpppG.

For example, a kinased ribozyme is contacted with guanosine triphosphate and guanyltransferase to add a m³G cap to the ribozyme. After such synthesis, the ribozyme can be gel purified using standard procedure. To ensure that the ribozyme has the desired activity, it may be tested with and without the 5' cap using standard procedures to assay both its enzymatic activity and its stability.

Synthetic ribozymes, including those containing various modifiers, can be purified by high pressure liquid chromatography (HPLC). Other liquid chromatography techniques, employing reverse phase columns and anion exchangers on silica and polymeric supports may also be used.

There follows an example of the synthesis of one ribozyme. A solid phase phosphoramidite chemistry is employed. Monomers used are 2'-*tert*-butyl-dimethylsilyl cyanoethylphosphoramidites of uridine, N-benzoyl-cytosine, N-phenoxyacetyl adenosine and guanosine (Glen Research, Sterling, VA). Solid phase synthesis is carried out on either an ABI 394 or 380B DNA/RNA synthesizer using the standard protocol provided with each machine. The only exception is that the coupling step is increased from 10 to 12 minutes. The phosphoramidite concentration is 0.1 M. Synthesis is done on a 1 µmole scale using a 1 µmole RNA reaction column (Glen Research). The average coupling efficiencies are between 97% and 98% for the 394 model, and between 97% and 99% for the 380B model, as determined by a calorimetric measurement of the released trityl cation.

Blocked ribozymes are cleaved from the solid support (e.g., CPG), and the bases and diphosphoester moiety deprotected in a sterile vial by dry ethanolic ammonia (2 mL) at 55°C for 16 hours. The reaction mixture is cooled on dry ice. Later, the cold liquid is transferred into a sterile screw cap vial and lyophilized. These conditions are suitable for removal of carbamate blocking groups in the 2' modifications shown in Formula I, and the amido and peptido linkages remain intact.

To remove the 2'-*tert*-butyl-dimethylsilyl groups from the ribozyme, the residue is suspended in 1 M tetra-n-butylammonium fluoride in dry THF (TBAF), using a 20-fold excess of the reagent for every silyl group, for 16 hours at ambient temperature (about 15-25°C). The reaction is quenched by adding an equal volume of sterile 1 M triethylamine acetate, pH 6.5. The sample is cooled and concentrated on a SpeedVac® to half the initial volume.

The ribozymes are purified in two steps by HPLC on a C4 300 Å 5 mm DeltaPak® column in an acetonitrile gradient.

The first step, or "trityl on" step, is a separation of 5'-DMT-protected ribozyme(s) from failure sequences lacking a 5'-DMT group. Solvents used for this step are: A (0.1 M triethylammonium acetate, pH 6.8) and B (acetonitrile). The elution profile is: 20% B for 10 minutes, followed by a linear gradient of 20% B to 50% B over 50 minutes, 50% B for 10 minutes, a linear gradient of 50% B to 100% B over 10 minutes, and a linear gradient of 100% B to 0% B over 10 minutes.

The second step is a purification of a completely deblocked ribozyme by a treatment of 2% trifluoroacetic acid on a C4 300 Å 5 mm DeltaPak® column in an acetonitrile gradient. Solvents used for this second step are: A (0.1 M Triethylammonium acetate, pH 6.8) and B (80% acetonitrile, 0.1 M triethylammonium acetate, pH 6.8). The elution profile is: 5% B for 5 minutes, a linear gradient of 5% B to 15% B over 60 minutes, 15% B for 10 minutes, and a linear gradient of 15% B to 0% B over 10 minutes.

The fraction containing ribozyme is cooled and lyophilized on a SpeedVac®. Solid residue is dissolved in a minimum amount of ethanol and sodium perchlorate in acetone. The ribozyme is collected by centrifugation, washed three times with acetone, and lyophilized.

### Administration of Ribozyme

Selected ribozymes can be administered prophylactically, or to diseased patients, e.g., by exogenous delivery of the ribozyme to an infected tissue by means of an appropriate delivery vehicle, e.g., a liposome, a controlled release vehicle, by use of iontophoresis, electroporation or ion paired molecules, or covalently attached adducts, and other pharmacologically approved methods of delivery. Routes of administration include intramuscular, aerosol, oral (tablet or pill form), topical, systemic, ocular, intraperitoneal and/or intrathecal. Expression vectors for immunization with ribozymes and/or delivery of ribozymes are also suitable.

The specific delivery route of any selected ribozyme will depend on the use of the ribozyme. Generally, a specific delivery program for each ribozyme will focus on naked ribozyme uptake with regard to intracellular localization, followed by demonstration of efficacy. Alternatively, delivery to these same cells in an organ or tissue of an animal can be pursued. Uptake studies will include uptake assays to evaluate cellular oligonucleotide uptake, regardless of the delivery vehicle or strategy. Such assays will also determine the intracellular localization of the ribozyme following uptake, ultimately establishing the requirements for maintenance of steady-state concentrations within the cellular compartment containing the target sequence (nucleus and/or cytoplasm). Efficacy and cytotoxicity can then be tested. Toxicity will not only include cell viability but also cell function.

Some methods of delivery that may be used include:
a. encapsulation in liposomes,
b. transduction by retroviral vectors,
c. conjugation with cholesterol,
d. localization to nuclear compartment utilizing antigen binding site found on most snRNAs,
e. neutralization of charge of ribozyme by using nucleotide derivatives, and
f. use of blood stem cells to distribute ribozymes throughout the body.

At least three types of delivery strategies are useful in the present invention, including: ribozyme modifications, particle carrier drug delivery vehicles, and retroviral expression vectors. Unmodified ribozymes, like most small molecules, are taken up by cells, albeit slowly. To enhance cellular uptake, the ribozyme may be modified essentially at random, in ways which reduces its charge but maintains specific functional groups. This results in a molecule which is able to diffuse across the cell membrane, thus removing the permeability barrier.

Modification of ribozymes to reduce charge is just one approach to enhance the cellular uptake of these larger molecules. The random approach, however, is not advisable since ribozymes are structurally and functionally more complex than small drug molecules. The structural requirements necessary to maintain ribozyme catalytic activity are well understood by those in the art. These requirements are taken into consideration when designing modifications to enhance cellular delivery. The modifications are also designed to reduce susceptibility to nuclease degradation. Both of these characteristics should greatly improve the efficacy of the ribozyme. Cellular uptake can be increased by several orders of magnitude without having to alter the phosphodiester linkages necessary for ribozyme cleavage activity.

Chemical modifications of the phosphate backbone will reduce the negative charge allowing free diffusion across the membrane. This principle has been successfully demonstrated for antisense DNA technology. The similarities in chemical composition between DNA and RNA make this a feasible approach. In the body, maintenance of an external concentration will be necessary to drive the diffusion of the modified ribozyme into the cells of the tissue. Administration routes which allow the diseased tissue to be exposed to a transient high concentration of the drug, which is slowly dissipated by systemic adsorption are preferred. Intravenous administration with a drug carrier designed to increase the circulation half-life of the ribozyme can be used. The size and composition of the drug carrier restricts rapid clearance from the blood stream. The carrier, made to accumulate at the site of infection, can protect the ribozyme from degradative processes.

Drug delivery vehicles are effective for both systemic and topical administration. They can be designed to serve as a slow release reservoir, or to deliver their contents directly to the target cell. An advantage of using direct delivery drug vehicles is that multiple molecules are delivered per uptake. Such vehicles have been shown to increase the circulation half-life of drugs which would otherwise be rapidly cleared from the blood stream. Some examples of such specialized drug delivery vehicles which fall into this category are liposomes, hydrogels, cyclodextrins, biodegradable nanocapsules, and bioadhesive microspheres.

From this category of delivery systems, liposomes are preferred. Liposomes increase intracellular stability, increase uptake efficiency and improve biological activity.

Liposomes are hollow spherical vesicles composed of lipids arranged in a similar fashion as those lipids which make up the cell membrane. They have an internal aqueous space for entrapping water soluble compounds and range in size from 0.05 to several microns in diameter. Several studies have shown that liposomes can deliver RNA to cells and that the RNA remains biologically active.

For example, a liposome delivery vehicle originally designed as a research tool, Lipofectin, has been shown to deliver intact mRNA molecules to cells yielding production of the corresponding protein.

Liposomes offer several advantages: They are non-toxic and biodegradable in composition; they display long circulation half-lives; and recognition molecules can be readily attached to their surface for targeting to tissues. Finally, cost effective manufacture of liposome-based pharmaceuticals, either in a liquid suspension or lyophilized product, has demonstrated the viability of this technology as an acceptable drug delivery system.

Other controlled release drug delivery systems, such as nonoparticles and hydrogels may be potential delivery vehicles for a ribozyme. These carriers have been developed for chemotherapeutic agents and protein-based pharmaceuticals, and consequently, can be adapted for ribozyme delivery.

Topical administration of ribozymes is advantageous since it allows localized concentration at the site of administration with minimal systemic adsorption. This simplifies the delivery strategy of the ribozyme to the disease site and reduces the extent of toxicological characterization. Furthermore, the amount of material to be applied is far less than that required for other administration routes. Effective delivery requires the ribozyme to diffuse into the infected cells. Chemical modification of the ribozyme to neutralize negative charge may be all that is required for penetration. However, in the event that charge neutralization is insufficient, the modified ribozyme can be co-formulated with permeability enhancers, such as Azone or oleic acid, in a liposome. The liposomes can either represent a slow release presentation vehicle in which the modified ribozyme and permeability enhancer transfer from the liposome into the infected cell, or the liposome phospholipids can participate directly with the modified ribozyme and permeability enhancer in facilitating cellular delivery. In some cases, both the ribozyme and permeability enhancer can be formulated into a suppository formulation for slow release.

Ribozymes may also be systemically administered. Systemic absorption refers to the accumulation of drugs in the blood stream followed by distribution throughout the entire body. Administration routes which lead to systemic absorption include: intravenous, subcutaneous, intraperitoneal, intranasal, intrathecal and ophthalmic. Each of these administration routes expose the ribozyme to an accessible diseased tissue. Subcutaneous administration drains into a localized lymph node which proceeds through the lymphatic network into the circulation. The rate of entry into the circulation has been shown to be a function of molecular weight or size. The use of a liposome or other drug carrier localizes the ribozyme at the lymph node. The ribozyme can be modified to diffuse into the cell, or the liposome can directly participate in the delivery of either the unmodified or modified ribozyme to the cell.

A liposome formulation which can deliver ribozymes to lymphocytes and macrophages is also useful for the initial site of influenza virus replication is in tissues of the nasopharynx and respiratory system. Coating of lymphocytes with liposomes containing ribozymes will target the ribozymes to infected cells expressing viral surface antigens. Whole blood studies show that the formulation is taken up by 90% of the lymphocytes after 8 hours at 37°C. Preliminary biodistribution and pharmacokinetic studies yielded 70% of the injected dose/gm of tissue in the spleen after one hour following intravenous administration.

Intraperitoneal administration also leads to entry into the circulation, with once again, the molecular weight or size controlling the rate of entry.

Liposomes injected intravenously show accumulation in the liver, lung and spleen. The composition and size can be adjusted so that this accumulation represents 30% to 40% of the injected dose. The rest is left to circulate in the blood stream for up to 24 hours.

The chosen method of delivery should result in cytoplasmic accumulation and molecules should have some nuclease-resistance for optimal dosing. Nuclear delivery may be used but is less preferable. Most preferred delivery methods include liposomes (10-400 nm), hydrogels, controlled-release polymers, microinjection or electroporation (for *ex vivo* treatments) and other pharmaceutically applicable vehicles. The dosage will depend upon the disease indication and the route of administration but should be between 100-200 mg/kg of body weight/day. The duration of treatment will extend through the course of the disease symptoms, usually at least 14-16 days and possibly continuously. Multiple daily doses are anticipated for topical applications, ocular applications and vaginal applications. The number of doses will depend upon disease delivery vehicle and efficacy data from clinical trials.

Establishment of therapeutic levels of ribozyme within the cell is dependent upon the rate of uptake and degradation. Decreasing the degree of degradation will prolong the intracellular half-life of the ribozyme. Thus, chemically modified ribozymes, e.g., with modification of the phosphate backbone, or capping of the 5' and 3' ends of the ribozyme with nucleotide analogues may require different dosaging. Descriptions of useful systems are provided in the art cited above, all of which is hereby incorporated by reference herein.

The claimed ribozymes are also useful as diagnostic tools to specifically or non-specifically detect the presence of a target RNA in a sample. That is, the target RNA, if present in the sample, will be specifically cleaved by the ribozyme, and thus can be readily and specifically detected as smaller RNA species. The presence of such smaller RNA species is indicative of the presence of the target RNA in the sample.

## Claims

1. The use of an oligonucleotide having enzymatic activity comprising a nucleotide having the formula: 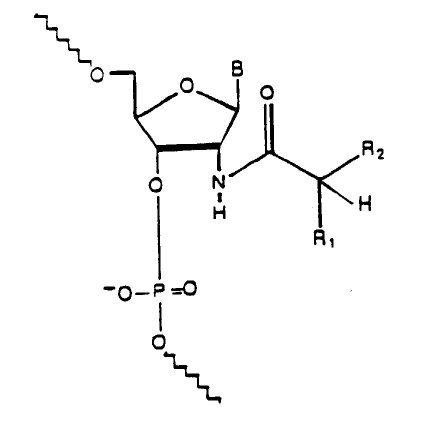 wherein B is a nucleotide base or hydrogen; R₁ and R₂ independently is selected from the group consisting of hydrogen, an alkyl group containing between 2 and 10 carbon atoms inclusive, an amine, an amino acid, and a peptide containing between 2 and 5 amino acids inclusive; and the zigzag lines are independently hydrogen or a bond in the manufacture of a medicament for cleavage of a target gene.

2. The use of an oligonucleotide according to claim 1 wherein said oligonucleotide is able to repeatedly cleave in a nucleotide base sequence specific manner.

3. The use of an oligonucleotide according to claim 1 or claim 2 wherein the oligonucleotide contains a phosphorothioate or phosphorodithioate internucleotide linkage.

4. The use of an oligonucleotide according to claim 1 wherein the oligonucleotide is formed in a hammerhead motif.

5. An oligonucleotide comprising a nucleotide having the formula: 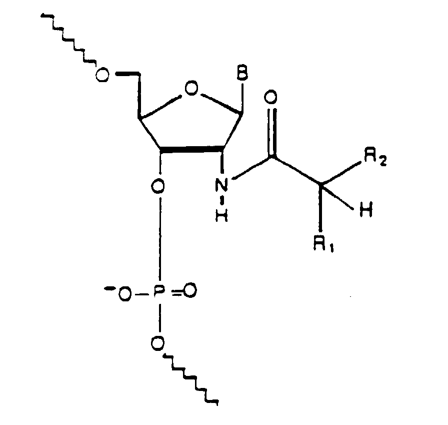 wherein B is a nucleotide base or hydrogen; R₁ and R₂ independently is selected from the group consisting of hydrogen, an alkyl group containing between 2 and 10 carbon atoms inclusive, an amine, an amino acid, and a peptide containing between 2 and 5 amino acids inclusive; and the zigzag lines are independently hydrogen or a bond;
provided that when B is uracil R₁ and R₂ are not both H or, when B is adenine one of R₁ and R₂ is NH₂ the other is not -CH₂-CH₂-COOH or, when B is uracil or adenine one of R₁ and R₂ is NH₂ the other is not H.

6. An oligonucleotide according to claim 5 wherein the oligonucleotide comprises an enzymatic RNA molecule.

7. An oligonucleotide according to claim 6 wherein said oligonucleotide is able to repeatedly cleave in a nucleotide base sequence specific manner.

8. An oligonucleotide according to claim 5 or claim 6 wherein the oligonucleotide contains a phosphorothioate or phosphorodithioate internucleotide linkage.

9. An oligonucleotide according to claim 6 wherein the oligonucleotide is formed in a hammerhead motif.

## Patentansprüche

1. Verwendung eines Oligonukleotids mit einer enzymatischen Aktivität, wobei ein Nukleotid mit der Formel beteiligt ist: 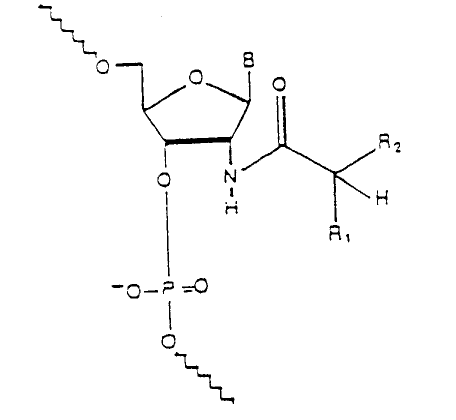 wobei B eine Nukleotidbase oder Wasserstoff ist; R₁ und R₂ unabhängig voneinander aus einer Gruppe ausgewählt sind, zu der Wasserstoff, eine Alkylgruppe mit zwischen 2 und 10 Kohlenstoffatomen einschließlich, ein Amin, eine Aminosäure und ein Peptid gehören, das zwischen 2 und 5 Aminosäuren enthält; und die Zickzacklinien unabhängig voneinander Wasserstoff oder eine Bindung bei der Herstellung eines Medikaments zum Spalten eines Zielgens darstellt.

2. Verwendung eines Oligonukleotids nach Anspruch 1, bei der das Oligonukleotid in der Lage ist, wiederholt in einer für eine Nukleotidbasissequenz spezifischen Weise zu spalten.

3. Verwendung eines Oligonukleotids nach einem der Ansprüche 1 oder 2, bei der das Oligonukleotid eine Phosphorthioat- oder eine Phosphordithioatinternukleotidbindung enthält.

4. Verwendung eines Oligonukleotids nach Anspruch 1, wobei das Oligonukleotid das Bild einea Hammerkopfes bildet.

5. Oligonukleotid mit der Formel: 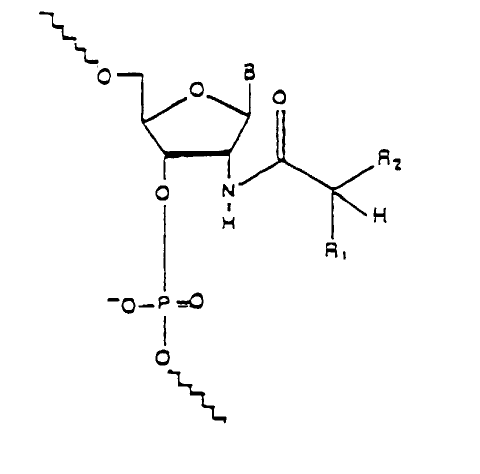 wobei B eine Nukleotidbase oder Wasserstoff ist; R₁ und R₂ unabhängig voneinander aus einer Gruppe ausgewählt sind, zu der Wasserstoff, eine Alkylgruppe mit zwischen 2 und 10 Kohlenstoffatomen einschließlich, ein Amin, eine Aminosäure und ein Peptid mit 2 und 5 Aminosäuren gehören; und die Zickzacklinien unabhängig voneinander Wasserstoff oder eine Bindung bedeuten;
vorausgesetzt, dass, wenn B Uracil ist, R₁ und R₂ nicht beide H sind oder, wenn B Adenin ist, entweder R₁ oder R₂ NH₂ ist, während der andere nicht -CH₂-CH₂-COOH ist oder, wenn B Uracil oder Adenin ist, entweder R₁ oder R₂ NH₂ und der andere H ist.

6. Oligonukleotid nach Anspruch 5, wobei das Oligonukleotid ein enzymatisches RNA-Molekül aufweist.

7. Oligonukleotid nach Anspruch 6, wobei das Oligonukleotid in der Lage ist, in einer für die Nukleotidbasensequenz spezifischen Weise wiederholt zu spalten.

8. Oligonukleotid nach Anspruch 5 oder 6, wobei das Oligonukleotid eine Phosphorthioat- oder eine Phosphordithioat-Internukleotidbindung aufweist.

9. Oligonukleotid nach Anspruch 6, wobei das Oligonukleotid das Bild eines Hammerkopfes bildet.

## Revendications

1. Utilisation d'un oligonucléotide doué d'activité enzymatique, comprenant un nucléotide ayant la formule : 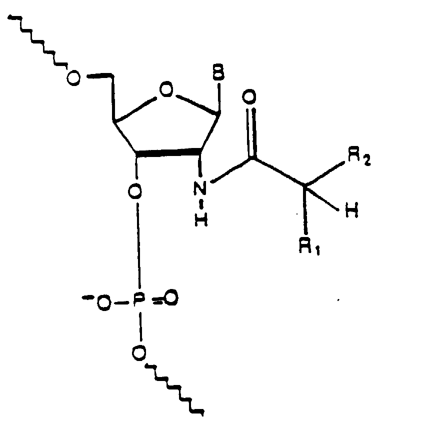 dans laquelle B est une base nucléotidique ou un atome d'hydrogène ; R₁ et R₂ sont choisis indépendamment dans la classe formée par un atome d'hydrogène, un groupe alkyle contenant 2 à 10 atomes de carbone inclusivement, une amine, un acide aminé et un peptide contenant 2 à 5 acides aminés inclusivement ; et les lignes en zigzag représentent indépendamment un atome d'hydrogène ou une liaison, dans la fabrication d'un médicament destiné au clivage d'un gène cible.

2. Utilisation d'un oligonucléotide selon la revendication 1, dans laquelle ledit oligonucléotide est capable d'effectuer un clivage à plusieurs reprises d'une manière spécifique envers une séquence de bases nucléotidiques.

3. Utilisation d'un oligonucléotide selon la revendication 1 ou la revendication 2, dans laquelle l'oligonucléotide contient une liaison internucléotidique thiophosphate ou dithiophosphate.

4. Utilisation d'un oligonucléotide selon la revendication 1, dans laquelle l'oligonucléotide a une configuration en tête de marteau.

5. Oligonucléotide comprenant un nucléotide ayant la formule : 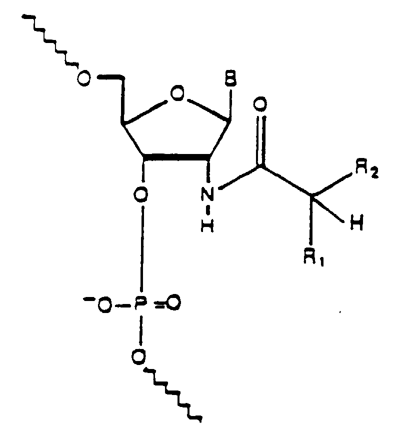 dans laquelle B est une base nucléotidique ou un atome d'hydrogène ; R₁ et R₂ sont choisis indépendamment dans la classe formée par un atome d'hydrogène, un groupe alkyle contenant 2 à 10 atomes de carbone inclusivement, une amine, un acide aminé et un peptide contenant 2 à 5 acides aminés inclusivement ; et les lignes en zigzag représentent indépendamment un atome d'hydrogène ou une liaison ; à condition que si B est l'uracile, R₁ et R₂ ne soient pas tous deux H, ou si B est l'adénine, l'un de R₁ et R₂ soit NH₂ et l'autre ne soit pas -CH₂-CH₂-COOH, ou si B est l'uracile ou l'adénine, l'un de R₁ et R₂ soit NH₂ et l'autre ne soit pas H.

6. Oligonucléotide selon la revendication 5, dans lequel l'oligonucléotide comprend une molécule d'ARN enzymatique.

7. Oligonucléotide selon la revendication 6, dans lequel ledit oligonucléotide est capable d'effectuer un clivage à plusieurs reprises d'une manière spécifique envers une séquence de bases nucléotidiques.

8. Oligonucléotide selon la revendication 5 ou la revendication 6, dans lequel l'oligonucléotide contient une liaison internucléotidique thiophosphate ou dithiophosphate.

9. Oligonucléotide selon la revendication 6, dans lequel l'oligonucléotide a une configuration en tête de marteau.
